(19) Europäisches Patentamt

European Patent Office

Office européen des brevets

(11) **EP 0 966 324 B1**

(12) **EUROPÄISCHE PATENTSCHRIFT**

(45) Veröffentlichungstag und Bekanntmachung des Hinweises auf die Patenterteilung:
**23.04.2003   Patentblatt 2003/17**

(51) Int Cl.7: **B01J 37/02**, C07C 51/255

(21) Anmeldenummer: **98909449.5**

(86) Internationale Anmeldenummer:
**PCT/EP98/00778**

(22) Anmeldetag: **12.02.1998**

(87) Internationale Veröffentlichungsnummer:
**WO 98/037967 (03.09.1998 Gazette 1998/35)**

(54) **VERFAHREN ZUR HERSTELLUNG VON SCHALENKATALYSATOREN FÜR DIE KATALYTISCHE GASPHASENOXIDATION VON AROMATISCHEN KOHLENWASSERSTOFFEN**

METHOD FOR PRODUCING SHELL CATALYSTS FOR CATALYTIC GAS-PHASE OXIDATION OF AROMATIC HYDROCARBONS

PROCEDE DE PREPARATION DE CATALYSEURS SOUS FORME DE COQUE POUR L'OXYDATION CATALYTIQUE EN PHASE GAZEUSE D'HYDROCARBURES AROMATIQUES

(84) Benannte Vertragsstaaten:
**DE FR IT**

(30) Priorität: **27.02.1997  DE 19707946**

(43) Veröffentlichungstag der Anmeldung:
**29.12.1999   Patentblatt 1999/52**

(73) Patentinhaber: **BASF AKTIENGESELLSCHAFT
67056 Ludwigshafen (DE)**

(72) Erfinder:
• **HEIDEMANN, Thomas
  D-69469 Weinheim (DE)**
• **CIMNIAK, Thomas
  D-67067 Ludwigshafen (DE)**
• **ULRICH, Bernhard
  D-67278 Bockenheim (DE)**

(56) Entgegenhaltungen:
**EP-A- 0 293 859          EP-A- 0 497 037
EP-A- 0 714 700          US-A- 3 926 846
US-A- 5 116 586**

**Beschreibung**

Die vorliegende Erfindung betrifft ein Verfahren zur Herstellung von Schalenkatalysatoren für die katalytische Gasphasenoxidation von aromatischen Kohlenwasserstoffen zu Carbonsäuren und/oder Carbonsäureanhydriden, auf deren Trägermaterial eine katalytisch aktive Metalloxide enthaltende Schicht schalenförmig aufgebracht ist.

Weiterhin betrifft die vorliegende Erfindung ein Verfahren für die katalytische Gasphasenoxidation von aromatischen Kohlenwasserstoffen zu Carbonsäuren und/oder Carbonsäureanhydriden mit einem molekularen Sauerstoff enthaltenden Gas in einem Festbett, bei erhöhter Temperatur und mittels einem oder mehreren, in Schichten im Reaktor angeordneten Schalenkatalysatoren, auf deren Trägermaterial eine Schicht aus katalytisch aktiven Metalloxiden schalenförmig aufgebracht ist.

Außerdem betrifft die vorliegende Erfindung Schalenkatalysatoren für die katalytische Gasphasenoxidation von aromatischen Kohlenwasserstoffen zu Carbonsäuren und/oder Carbonsäureanhydriden auf. deren Trägermaterial eine Schicht aus katalytisch aktiven Metalloxiden schalenförmig aufgebracht ist.

Bekanntermaßen wird eine Vielzahl von Carbonsäuren und/oder Carbonsäureanhydriden technisch durch die katalytische Gasphasenoxidation von aromatischen Kohlenwasserstoffen, wie Benzol, den Xylolen, Naphthalin, Toluol oder Durol, in Festbettreaktoren, vorzugsweise Rohrbündelreaktoren, hergestellt. Dabei werden beispielsweise Benzoesäure, Maleinsäureanhydrid, Phthalsäureanhydrid, Isophthalsäure, Terephthalsäure oder Pyromellithsäureanhydrid gewonnen. Dazu wird im allgemeinen ein Gemisch aus einem molekularen Sauerstoff enthaltenden Gas, beispielsweise Luft und das zu oxidierende Ausgangsmaterial durch eine Vielzahl in einem Reaktor angeordneter Rohre geleitet, in denen sich eine Schüttung mindestens eines Katalysators befindet. Zur Temperaturregelung sind die Rohre von einem Wärmeträgermedium, beispielsweise einer Salzschmelze, umgeben. Trotz dieser Thermostatisierung kann es in der Katalysatorschüttung zur Ausbildung sogenannter "Heißer Flekken" (hot spots) kommen, in denen eine höhere Temperatur herrscht als im übrigen Teil der Katalysatorschüttung. Diese "hot spots" geben Anlaß zu Nebenreaktionen, wie der Totalverbrennung des Ausgangsmaterials oder führen zur Bildung unerwünschter, vom Reaktionsprodukt nicht oder nur mit viel Aufwand abtrennbarer Nebenprodukte, beispielsweise zur Bildung von Phthalid oder Benzoesäure, bei der Herstellung von Phthalsäureanhydrid (PSA) aus o-Xylol. Des weiteren steht die Ausbildung eines ausgeprägten hot spots beim Anfahren einer Anlage einer zügigen Erhöhung der Beladung des dem Reaktor zugeführten Gases mit Xylol entgegen, da ab einer bestimmten not spot-Temperatur der Katalysator irreversibel geschädigt werden kann. Aus diesem Grunde kann die Xylolbeladung der Gaszufuhr beim Anfahren des Reaktors nur langsam, in kleinen Schritten auf die verfahrensgemäß vorgesehene Xylolbeladung erhöht werden, wobei diese schrittweise Beladungserhöhung sehr sorgfältig kontrolliert werden muß.

Zur Verringerung des Ausmaßes der durch hot spots-verursachten Nebenreaktionen wurde in der Technik dazu übergegangen, unterschiedlich aktive Katalysatoren schichtweise in der Katalysatorschüttung anzuordnen, wobei in der Regel der weniger aktive Katalysator so im Festbett angeordnet ist, daß das Reaktionsgasgemisch mit ihm als erstes in Kontakt kommt, d.h. er liegt in der Schüttung zum Gaseintritt hin, wohingegen der aktivere Katalysator zum Gasaustritt aus der Katalysatorschüttung hin gelegen ist. Die unterschiedlich aktiven Katalysatoren in der Katalysatorschüttung können bei der gleichen Temperatur dem Reaktionsgas ausgesetzt werden, es können die beiden Schichten aus unterschiedlich aktiven Katalysatoren aber auch auf unterschiedliche Reaktionstemperaturen thermostatisiert mit dem Reaktionsgas in Kontakt gebracht werden.

Als Katalysatoren haben sich für diese Oxidationsreaktionen sogenannte Schalenkatalysatoren bewährt, bei denen die katalytisch aktive Masse schalenförmig auf einem im allgemeinen unter den Reaktionsbedingungen inerten Trägermaterial, wie Quarz ($SiO_2$), Porzellan, Magnesiumoxid, Zinndioxid, Siliciumcarbid, Rutil, .Tonerde ($Al_2O_3$), Aluminiumsilikat, Magnesiumsilikat (Steatit), Zirkoniumsilikat oder Cersilikat oder Mischungen dieser Trägermaterialien aufgebracht ist. Als katalytisch aktiver Bestandteil der katalytisch aktiven Masse dieser Schalenkatalysatoren dient im allgemeinen neben Titandioxid in Form seiner Anatasmodifikation Vanadiumpentoxid. Des weiteren können in der katalytisch aktiven Masse in geringen Mengen eine Vielzahl anderer oxidischer Verbindungen enthalten sein, die als Promotoren die Aktivität und Selektivität des Katalysators beeinflussen, beispielsweise in dem sie seine Aktivität absenken oder erhöhen. Als solche Promotoren seien beispielhaft die Alkalimetalloxide, insbesondere Lithium-, Kalium-, Rubidium-und Cäsiumoxid, Thallium(I)oxid, Aluminiumoxid, zirkoniumoxid, Eisenoxid, Nickeloxid, Kobaltoxid, Manganoxid, Zinnoxid, Silberoxid, Kupferoxid, Chromoxid, Molybdänoxid, Wolframoxid, Iridiumoxid, Tantaloxid, Nioboxid, Arsenoxid, Antimonoxid, Ceroxid und Phosphorpentoxid genannt. Als die Aktivität vermindernder und die Selektivität erhöhender Promotor wirken z.B. die Alkalimetalloxide, wohingegen oxidische Phosphor-verbindungen, insbesondere Phosphorpentoxid die Aktivität des Katalysators erhöhen, aber dessen Selektivität vermindern.

Zur Herstellung derartiger Schalenkatalysatoren wird nach den Verfahren von DE-A 1642938 und DE-A 1769998 eine wäßrige und/oder ein organisches Lösungsmittel enthaltende Lösung oder Suspension der Aktivmassenbestandteile und/oder deren Vorläuferverbindungen, welche im folgenden als "Maische" bezeichnet wird, auf das Trägermaterial in einer beheizten Dragiertrommel bei erhöhter Temperatur aufgesprüht, bis der gewünschte Aktivmassenanteil am Katalysatorgesamtgewicht erreicht ist. Beim Aufsprühen treten allerdings hohe Verluste auf, da erhebliche Mengen der Maische vernebelt und durch das Abgas aus der Dragiertrommel ausgetragen werden. Da der Aktivmasseanteil am Gesamtkatalysator im allgemeinen nur eine geringe Abweichung vom Sollwert haben soll, da durch die Menge der aufgebrachten Aktivmasse und die Schichtdicke der Schale Aktivität und Selektivität des Katalysators stark beeinflußt werden, muß der Katalysator bei der geschilderten Herstellungsweise häufig zur Bestimmung der aufgebrachten Aktivmassemenge abgekühlt, aus der Dragiertrommel entnommen und nachgewogen werden. Wird zu viel Aktivmasse auf dem Katalysatorträger abgeschieden, ist im allgemeinen eine nachträgliche, schonende Entfernung der zu viel aufgetragenen Aktivmassemenge ohne Beeinträchtigung der Festigkeit der Schale, insbesondere ohne Rißbildung in der Katalysatorschale, nicht möglich.

Weitere für Oxidationsreaktionen wichtige Katalysatorparameter, nämlich die makroskopische Struktur der Aktivmasse, beispielsweise deren Porosität und Porenradienverteilung, sowie die Aktivmassenzusammensetzung innerhalb der Aktivmassenschale, lassen sich nach den oben erwähnten Verfahren nur sehr. schwierig gezielt beeinflussen. Die in DE-A 2212964 beschriebene Methode der sequentiellen Aufsprühung von Maischen verschiedener Zusammensetzung führt nicht zu definierten Aktivmassenzusammensetzungen innerhalb der Aktivmassenschale, da Chromatographieeffekte, die während der Aufbringung in der beheizten Dragiertrommel auftreten, zur unkontrollierbaren Ausbildung von Konzentrationsgradienten der Aktivstoffe in der Katalysatorschale führen, die eine uneinheitliche Zusammensetzung der Aktivmasse in der Katalysatorschale und in den einzelnen Katalysatorpartikeln zur Folge haben.

Gasphasenoxidationen an den oben geschilderten Schalenkatalysatoren finden nicht allein an der äußeren Oberfläche der Schale statt. Zur Erzielung einer für die vollständige Umsetzung der in technischen Verfahren angewandten hohen Beladungen des Reaktions-gases mit Edukt erforderlichen Kätalysätoraktivität und selektivität ist eine effiziente Nutzung der gesamten Aktivmassenschale des Katalysators und damit eine gute Zugänglichkeit des Reaktionsgases zu den in dieser Schale gelegenen Reaktionszentren notwendig. Da die Oxidation aromatischer Verbindungen zu Carbonsäuren und/oder Carbonsäureanhydriden über eine Vielzahl von Zwischenstufen vor sich geht und das Wertprodukt am Katalysator zu Kohlendioxid und Wasser weiteroxidiert werden kann, ist zur Erzielung eines hohen Umsatzes an Edukt unter gleichzeitiger Zurückdrängung des oxidativen Abbaus des Wertprodukts eine optimale Anpassung der Verweilzeit des Reaktionsgases in der Aktivmasse durch die Erzeugung einer geeigneten, makroskopischen Aktivmassestruktur in der Kätalysatorschale erforderlich.

Des weiteren ist zu berücksichtigen, daß die Gaszusammensetzung an der äußeren Oberfläche der Aktivmassenschale nicht zwangsläufig auch der Gaszusammensetzung im Inneren der Aktivmasse entspricht. Vielmehr ist zu erwarten, daß die Konzentration an primären Oxidationsprodukten höher, die Edukt-Konzentration dagegen niedriger ist, als an der äußeren Katalysatoroberfläche. Dieser unterschiedlichen Gaszusammensetzung sollte durch eine gezielte Einstellung der Aktivmassenzusammensetzung innerhalb der Aktivmassenschale Rechnung getragen werden, um zu einer optimalen Katalysatoraktivität und -selektivität zu gelangen.

EP-A 714 700 betrifft ein Verfahren zur Herstellung eines Katalysators, der aus einem Trägerkörper und einer auf der Oberfläche des Trägerkörpers aufgebrachten, katalytisch aktiven Oxidmasse besteht, bei dem man den Trägerkörper mit einer wäßrigen Lösung einer bei Normaldruck oberhalb von 100°C siedenden, organischen Substanz als Haftflüssigkeit zunächst befeuchtet und danach durch in Kontakt bringen mit trockener, feinteiliger aktiver Oxidmasse an der Oberfläche des befeuchteten Trägerkörpers eine Schicht aktiver Oxidmasse anheftet und anschließend die Haftflüssigkeit aus dem mit aktiver Oxidmasse beschichteten, befeuchteten Trägerkörper entfernt. Dieses Verfahren hat den Nachteil, daß es relativ hohen Arbeits- und Zeitaufwand erfordert und die Abriebfestigkeit der hergestellten Katalysatoren verbesserungswürdig ist.

DE-A 40 38 109 betrifft ein Verfahren zur Herstellung von Formkörpern mit poröser Oberfläche und enger Oberflächenporenradienverteilung, sowie die Verwendung dieser Formkörper als Trägerkörper für die Fixierung bzw. Immobilisierung von Indikatoren, von Katalysatoren, von Biomasse oder von Teilen der Biomasse. Die Formkörper werden mit kleinen, gleichförmigen Partikeln mit enger Partikelgrößenverteilung eines agglomerationsfähigen Materials ohne Anwendung von Druck, ohne Zusatz eines Bindemittels und bei Temperaturen unterhalb von 120°C beschichtet, so daß die zwischen den kleinen, gleichförmigen Partikeln an der. Oberfläche mit einer engen Porenradienverteilung bilden. Die Form und die Größe der Porenradien wird dabei durch die Größe der kleinen, gleichförmigen Partikel festgelegt, und die Porenradienverteilung wird durch die Partikelgrößenverteilung bestimmt. Dieses Verfahren hat ähnliche Nachteile, wie sie im Hinblick auf EP-A 714 700 dargelegt wurden. Darüber hinaus halten die auf diese Weise beschichteten Formkörper den technischen und mechanischen Belastungen großtechnischer Verfahren zur Oxidation aromatischer Verbindungen nicht stand, wodurch sich die mechanische Stabilität im Dauerbetrieb verändert und ebenso die katalytischen Eigenschaften infolge thermischen Sinterns der aufgetragenen Beschichtungsmasse. DE-A 40 38 109 enthält dementsprechend auch keine Ausführungen über die Anwendbarkeit der nach deren Verfahren beschichteten Formkörper in Verfahren zur großtechnischen Oxidation von aromatischen Kohlenwasserstoffen.

EP-A 293 859 und US-A 3 926 846 offenbaren ein Verfahren zur HerStellung von Schalenkatalysatoren, bei dem aus einer Lösung und/oder Suspension der katalytisch aktiven Metalloxide und deren Vorläuferverbindungen durch Sprühtrocknung oder Gefriertrocknung ein Pulver hergestellt wird, das anschließend als Paste auf den Träger, ohne vorausgegangene Wärmebehandlung, aufgebracht wird.

Der vorliegenden Erfindung lag somit die Aufgabe zugrunde, ein Verfahren zur Herstellung von Schalenkatalysatoren für die katalytische Gasphasenoxidation von aromatischen Kohlenwasserstoffen zu Carbonsäuren und/ oder Carbonsäureanhydriden zu finden, das sowohl die Vermeidung der geschilderten Nachteile des Aufsprühverfahrens sowie anderer Beschichtungsverfahren des Standes der Technik als auch die Auftragung einer in engen Grenzen vorgegebenen Aktivmassemenge auf einfache Weise ermöglicht. Des weiteren sollte durch dieses Verfahren die makroskopische Aktivmassestruktur, also z.B. deren Porosität und Porenradienverteilung, als auch die Aktivmassezusammensetzung über die Dicke der Katalysatorschale beeinflußbar sein und es auf diese Weise ermöglicht werden, Katalysatoren mit verbesserten Eigenschaften, wie verbesserter mechanischer Stabilität und/oder verbesserter Aktivität und/oder Selektivität, insbesondere auch während der Anfahrphase bis zur Erreichung des Vollastbetriebes einer Anlage zur Gasphasenoxidation von aromatischen Verbindungen, herzustellen.

Dementsprechend wurde ein Verfahren zur Herstellung von Schalen-katalysatoren für die katalytische Gasphasenoxidation von aromatischen Kohlenwasserstoffen zu Carbonsäuren und/oder Carbonsäureanhydriden, auf deren Trägermaterial eine katalytisch aktive Metalloxide enthaltende Schicht schalenförmig aufgebracht ist, gefunden, das dadurch gekennzeichnet ist, daß aus einer Lösung und/oder einer Suspension der katalytisch aktiven Metalloxide und deren Vorläuferverbindungen oder diesen Vorläuferverbindungen alleine in An- oder Abwesenheit von Hilfsmitteln für die Katalysatorherstellung durch Sprühtrocknung oder Gefriertrocknung zunächst ein Pulver hergestellt wird, das anschließend als Paste in An- oder Abwesenheit von Hilfsmitteln für die Katalysatorherstellung auf dem Träger nach oder ohne vorausgegangene Konditionierung, ohne vorausgegangene Wärmebehandlung zur Erzeugung der katalytisch aktiven Metalloxide schalenförmig aufgebracht und der auf diese Weise beschichtete Träger einer Wärmebehandlung zur Erzeugung der katalytisch aktiven Metalloxide unterzogen wird, wobei die durchschnittliche Schichtdicke der Aktivmasse der so hergestellten Schalenkatalysatoren 10 bis 250 µm beträgt.

Des weiteren betrifft die vorliegende Erfindung ein Verfahren zur katalytischen Gasphasenoxidation von aromatischen Kohlenwasserstoffen zu Carbonsäuren und/oder Carbonsäureanhydriden, in dem die erfindungsgemäß hergestellten Katalysatoren verwendet werden, sowie die erfindungsgemäß hergestellten Katalysatoren.

Die Aufgabe der Erfindung wird somit, kurz gefaßt, dadurch gelöst, daß aus der Maische zunächst ein Vorläuferverbindungen der katalytisch aktiven Metalloxide enthaltendes Pulver erzeugt wird, mit dem anschließend der Katalysatorträger gegebenenfalls unter Verwendung von Hilfsmitteln beschichtet wird. Die Beschichtung des Trägers mit einem ausschließlich, katalytisch aktive Metalloxide, jedoch keine Vorläuferverbindungen dieser Metalloxide enthaltenden Pulver ist somit nicht erfindungsgemäß.

Das zur Beschichtung des Katalysatorträgers dienende, die katalytisch aktiven Metalloxide und deren Vorläuferverbindungen oder diese Vorläuferverbindungen alleine enthaltende Pulver kann aus der diese Bestandteile enthaltenden Maische nach verschiedenen Verfahren, beispielsweise durch Sprühtrocknung oder Gefriertrocknung, vorzugsweise durch Sprühtrocknung, hergestellt werden. In der Maische können die katalytisch aktiven Metalloxide und deren Vorläuferverbindungen oder diese Vorläuferverbindungen alleine als feste Partikel suspendiert sein oder gelöst vorliegen. Vorzugsweise enthält die Maische diese katalytisch aktiven Komponenten und deren Vorläuferverbindungen oder diese Vorläuferverbindungen alleine in den Mengenverhältnissen, wie sie später auch in der katalytisch aktiven Masse des fertigen Katalysators vorliegen sollen. Es ist aber auch möglich, dem erfindungsgemäß hergestellten, zur Beschichtung des Katalysatorträgers dienenden Pulver nach dessen Herstellung einzelne Bestandteile der.katalytisch aktiven Masse nachträglich hinzuzufügen. Außer den katalytisch aktiven Aktivmassebestandteilen und deren Vorläuferverbindungen oder diesen Vorläuferverbindungen alleine kann die Maische Hilfsmittel für die Katalysatorherstellung, beispiels- . weise Bindemittel und/oder Porenbildner, enthalten.

Zur Herstellung der Maische werden die Aktivmassebestandteile des Katalysators beispielsweise in Form ihrer Oxide, Salze, beispielsweise ihrer Nitrate, $C_1$-bis $C_{10}$-Carboxylate, Carbonate, Hydrogencarbonate, Sulfate, Hydrogensulfate, Halogenide oder Phosphate oder als Komplexverbindungen, beispielsweise als Oxalat- oder Acetylaceton-Komplex sowie die gegebenenfalls zur Katalysatorherstellung eingesetzten Hilfsmittel in einem Lösungsmittel gelöst oder, falls dieses Stoffe im einzelnen nicht löslich sind, suspendiert. Als Lösungs- oder Suspendierungsmittel können sowohl Wasser als auch organische Flüssigkeiten oder Mischungen des Wassers mit diesen Flüssigkeiten verwendet werden. Vorzugsweise wird Wasser oder Wasser im Gemisch mit organischen Flüssigkeiten verwendet, wobei das Mischungsverhältnis Wasser/organische Flüssigkeit im allgemeinen zwar nicht kritisch ist, vorzugsweise aber solche Lösungsmittelgemische eingesetzt werden, die 50 oder mehr Gewichtsprozent Wasser enthalten.

Als organische Flüssigkeiten werden vorzugsweise wasserlösliche Lösungsmittel, wie $C_1$- bis $C_4$-Alkohole, wasserlösliche Ether, z.B. Tetrahydrofuran, Dioxan oder Ethylenglykoldimethylether, wasserlösliche Amide, z.B. Formamid, Pyrrolidon, N-Methylpyrrolidon, N,N-Dimethylformamid oder wasserlösliche Sulfoxide, z.B. Dimethylsulfoxid, verwendet.

Als Hilfsmittel für die Katalysatorherstellung können der Maische Bindemittel, Porenbildner und/oder temporäre Aktivitätsdämpfungsmittel zugesetzt werden.

Unter dem Begriff Bindemittel werden hierbei Substanzen verstanden, die die Haftung der Aktivmassepulverpartikel untereinander und/oder auf dem Trägermaterial dauerhaft oder temporär verbessern. Im erfindungsgemäßen Verfahren können als Bindemittel beispielsweise Polyole, wie Ethylenglykol, Propylenglykol, Butylenglykole oder Glycerin oder Amide, wie Formamid, N,N-Dimethylformamid, N,N-Diethylformamid, N,N-Dibutylformamid, Acetamid, Pyrrolidon oder N-Methylpyrrolidon verwendet werden.

Als Porenbildner werden im folgenden Substanzen bezeichnet, die in der Aktivmasse durch eine Volumenänderung, beispielsweise durch Verdampfung oder Zersetzung während der Wärmebehandlung zur Erzeugung der katalytisch aktiven Metalloxide aus deren Vorläuferverbindungen, bei der Herstellung des Schalenkatalysators, die Herausbildung einer im Vergleich zu einer Aktivmasse, bei deren Herstellung kein Porenbildner zugesetzt wurde, geänderte Porenstruktur bewirken. Als Porenbildner können im erfindungsgemäßen Verfahren beispielsweise Polyole, wie Glycerin oder Polymere, wie Cellulose, Methylcellulose, Celluloseacetat oder Stärke oder Säuren, wie Oxalsäure, Weinsäure oder Milchsäure oder auch Amine, wie Melamin oder Amide, wie Harnstoff eingesetzt werden. Die Art und die Menge der zuzusetzenden Hilfsmittel richtet sich im allgemeinen nach der chemischen zusammensetzung der herzustellenden Aktivmasse des Schalenkatalysators und wird zweckmäßigerweise für die jeweils herzustellende Aktivmasse bestimmter chemischer Zusammensetzung in einem Vorversuch optimiert.

Unter dem Begriff temporäre Aktivitätsdämpfungsmittel werden oben angegebene Bindemittel oder Porenbildner oder darüber hinaus auch alle weiteren Hilfsstoffe verstanden, die für einen begrenzten Zeitraum während der Anfahrphase des mit dem Katalysator beschickten Reaktors bis zu dessen Vollastbetrieb zu einer gezielten Aktivitätsverminderung und/oder zu einer Erniedrigung der hot spot-Temperaturen führen und somit die Anfahrprozedur bzw. die sich daran anschließende Beladungserhöhung des dem Katalysator zugeführten Gasstroms mit Edukt erleichtern ohne die mittlere katalytische Aktivität bzw. Selektivität zu vermindern. Diese temporären Aktivitätsdämpfungsmittel werden bei den Temperaturen, bei denen der Oxidationsreaktor während des Anfahrens und der Beladungserhöhung betrieben wird, nach und nach zersetzt und verlieren so nach und nach ihre die Aktivität des Katalysators vermindernde Wirkung. Als temporäre Akitvitätsdämpfungsmittel können z.B. Polyole, wie Ethylenglykol, Polymere, wie Cellulose oder Stärke, oder Amine, wie Melamin, im Katalysator enthalten sein.

Alternativ können diese Hilfsmittel auch dem aus der Maische erfindungsgemäß bereiteten Pulver zugemischt werden. Ebenso ist es möglich einen Teil der Hilfsmittel der Maische und den Rest dem aus der Maische erzeugten Pulver vor oder während der Beschichtung des Katalysatorträgers mit dem Maische-Pulver zuzumischen.

Zur Herstellung des die Aktivmassebestandteile und deren Vorläuferverbindungen oder diese Vorläuferverbindungen alleine enthaltenden Pulvers mittels Sprühtrocknung wird die Maische im allgemeinen zu kleinen Tröpfchen versprüht, aus denen die Flüssigkeit im Gleichstrom oder Gegenstrom mittels eines Stroms heißen Gases, wie Luft, Stickstoff, Kohlendioxid usw., vorzugsweise Luft, verdampft wird. Dabei können die getrockneten, pulverförmigen, die Aktivmassebestandteile und deren Vorläuferverbindungen oder diese Vorläuferverbindungen alleine enthaltenden Feststoffpartikel je nach konstruktiver Ausgestaltung der Sprühtrocknungsapparatur durch Schwerkraft oder durch Zentrifugalkraft, beispielsweise mit Zyklonen, abgeschieden werden. Zur Sprühtrocknung wird im allgemeinen eine Gaseintrittstemperatur des Trocknungsgases von 250 bis 450°C, vorzugsweise von 260 bis 350°C, eingestellt und die Zufuhr von Trocknungsgas und Maische in die Sprühtrocknungsapparatur so geregelt, daß das Trocknungsgas . die Sprühtrocknungsapparatur mit einer Temperatur von im allgemeinen 100 bis 200°C, vorzugsweise 110 bis 150°C verläßt. Praktischerweise wird die Sprühtrocknung bei Atmosphärendruck ausgeführt, es kann jedoch auch vorteilhaft bei vermindertem Druck getrocknet werden. Zur Sprühtrocknung können handelsübliche Sprühtrockner verwendet werden, beispielsweise Sprühtrockner der Firma A/S Niro Atomizer, Kopenhagen.

Alternativ zur Sprühtrocknung kann die Maische mittels Gefriertrocknung zu einem Pulver getrocknet werden. Dazu wird die Maische im allgemeinen auf beispielsweise mittels flüssigem Stickstoff oder flüssiger Luft tiefgekühlten Metallplatten oder -bändern tiefgefroren und das gefrorene Lösungsmittel aus der gefrorenen Maische bei vermindertem Druck sublimiert, wobei aus der Maische ein pulverförmiges Produkt entsteht. Zur Gefriertrocknung der Maische können handelsübliche Gefriertrocknungsanlagen, beispielsweise Gefriertrocknungsanlagen der Firma Leybold-Heraeus GmbH, Köln, eingesetzt werden.

Die durch Sprühtrocknung oder Gefriertrocknung erhaltenen Pulver können für die spätere Beschichtung des Katalysators gewünschtenfalls zusätzlich konditioniert werden, beispielsweise durch die Einstellung einer gewünschten Korngrößenverteilung der Pulverpartikel. Diese Konditionierung kann nach an sich üblichen Methoden, z.B. durch Sieben oder windsichten, durchgeführt werden. Gröbere Partikel können durch Mahlen, beispielsweise in Kugeloder Strahlmühlen, auf die gewünschte Korngröße zerkleinert werden.

Im allgemeinen wird das erfindungsgemäß hergestellte Maische-Pul-ver auf Korngrößen von 0,1 bis 100 $\mu$m, vorzugsweise von 0,5 bis 80 $\mu$m und besonders bevorzugt von 1,0 bis 60 $\mu$m konditioniert.

Mit dem auf die beschriebene Weise erzeugten Maischepulver, das die katalytisch aktiven Metalloxide und deren Vorläuferverbindungen oder diese Vorläuferverbindungen alleine sowie gegebenenfalls der Maische zugesetzte Hilfsmittel für die Katalysatorherstellung enthält, wird der Katalysatorträger in an sich herkömmlichen Beschichtungsvorrichtungen, beispielsweise Dragiertrommeln, beschichtet.

Als Trägermaterialien können praktisch alle Trägermaterialien des Standes der Technik, wie sie vorteilhaft bei der Herstellung von Schalenkatalysatoren für die Oxidation aromatischer Kohlenwasserstoffe zu Carbonsäuren und/oder Carbonsäureanhydriden eingesetzt werden, Verwendung finden, beispielsweise die eingangs bei der Schilderung des Standes der Technik erwähnten Trägermaterialien. Als vorteilhafte Trägermaterialien sind insbesondere Steatit und Siliciumcarbid zu nennen.

Die Form des zu beschichtenden Trägermaterials ist im allgemeinen für das erfindungsgemäße Katalysatorherstellungsverfahren nicht kritisch. Beispielsweise können Katalysatorträger in Form von Kugeln, Ringen, Tabletten, Extrudaten oder Splitt verwendet werden.

Vor der Beschichtung des Katalysatorträgers können dem Maische-Pulver Hilfsmittel für die Katalysatorherstellung, wie Bindemittel, Porenbildner oder temporäre Aktivitätsdämpfungsmittel, oder, falls diese Hilfsmittel nicht schon der Maische zugesetzt wurden, eine zusätzliche Menge dieser Hilfsmittel zugemischt werden. Dieser Zusatz kann auch noch während des Beschichtungsvorgangs erfolgen.

Zur Beschichtung des Katalysatorträgers wird das erfindungsgemäß hergestellte Maischepulver im allgemeinen mit Wasser und/oder einer organischen Flüssigkeit, zweckmäßigerweise einer organischen Flüssigkeit, die als Bindemittel wirkt, vorzugsweise nur mit Wasser, vermengt. Zusätzlich können der Flüssigkeit vor der Vermengung mit dem Maische-Pulver weitere Hilfsmittel zugesetzt werden. Die Zubereitung dieser Paste kann sowohl innerhalb der Beschichtungsvorrichtung in Gegenwart des Katalysatorträgers als auch separat, außerhalb der Beschichtungsvorrichtung in einer Mischvorrichtung, beispielsweise einer Rühr- oder Knetmaschine, erfolgen.

Die Beschichtung des Katalysatorträgers in der Beschichtungsvor-richtung wird im allgemeinen bei Temperaturen von 10 bis 50°C, vorzugsweise bei Raumtemperatur, vorgenommen.

Der beschichtete Katalysatorträger wird anschließend im allgemeinen bei Temperaturen von 20 bis 200°C, vorzugsweise von 20 bis 120°C getrocknet. Die Trocknung kann in der Beschichtungsvorrichtung, falls diese beheizbar ist, oder in an sich herkömmlichen Trocknern vorgenommen werden. Die Trocknung kann bei Atmosphärendruck oder unter vermindertem Druck ausgeführt werden.

Das erfindungsgemäße Katalysatorherstellungsverfahren ermöglicht es auch Schalenkatalysatoren herzustellen, in denen die die katalytisch aktiven Bestandteile enthaltende Schale aus zwei oder mehr Schichten mit definierter, unterschiedlicher Zusammensetzung an Aktivkomponenten aufgebaut ist. Hierzu wird zunächst der Katalysatorträger auf die vorstehend beschriebene Weise mit einer Schicht einer definierten Zusammensetzung aus den katalytisch aktiven Metalloxiden und deren Vorläuferverbindungen oder diesen Vorläuferverbindungen alleine beschichtet, anschließend diese erste Schicht vorteilhaft getrocknet und gewünschtenfalls einer Wärmebehandlung unterzogen und auf diese Schicht nach der gleichen Methodik eine zweite Schicht oder weitere Schichten aufgetragen.

Die durchschnittliche Schichtdicke der Aktivmasse der erfindungsgemäß hergestellten Schalenkatalysatoren zur Gasphasenoxidation aromatischer Verbindungen zu Carbonsäuren und/oder Carbonsäureanhydriden beträgt im allgemeinen 10 bis 250$\mu$m, vorzugsweise 50 bis 200$\mu$m und besonders bevorzugt 70 bis 150$\mu$m. Die Aktivmasse der erfindungsgemäß hergestellten Schalenkatalysatoren hat im allgemeinen eine BET-Oberfläche von 1 bis 100m$^2$/g, vorzugsweise von 5 bis 50m$^2$/g und besonders bevorzugt von 7 bis 30m$^2$/g, bestimmt nach dem Verfahren von Brunauer et al, J. Am. Chem. Soc. 60, 309 (1938). Das Porenvolumen der Aktivmasse, bestimmt nach dem Verfahren der Quecksilber-Porosimetrie, beträgt im allgemeinen 0,1 bis 2,5ml/g, vorzugsweise 0,2 bis 1,5 ml/g und besonders bevorzugt 0,3 bis 1,1ml/g für die Poren mit Porendurchmessern von 1,7 bis 400nm.

Das erfindungsgemäße Verfahren zur Herstellung von Schalenkatalysatoren zur Gasphasenoxidation von aromatischen Kohlenwasserstoffen zu Carbonsäuren und/oder Carbonsäureanhydriden kann praktisch zur Herstellung aller Schalenkatalysatoren für diesen Zweck benutzt werden. Insbesondere können mit dem erfindungsgemäßen Verfahren Schalenkatalysatoren mit besonders vorteilhaften Eigenschaften hergestellt werden, die in ihrer katalytisch aktiven Masse neben Titandioxid in seiner Anastasmodifikation, oxidische Verbindungen des Vanadiums, insbesondere Vanadiumpentoxid (V$_2$O$_5$), sowie gewünschtenfalls oxidische Promotoren, beispielsweise auf der Basis von Alkalimetallen, Erdalkalimetallen, Thallium, Aluminium, Zirkonium, Eisen, Nickel, Kobalt, Mangan, Zinn, Silber, Kupfer, Chrom, Molybdän, Wolfram, Iridium, Tantal, Niob, Arsen, Antimon, Cer und/oder von Phosphor enthalten und in denen die katalytisch aktive Masse schalenförmig auf einem Katalysatorträger aus z.B. Quarz, Porzellan, Magnesiumoxid, Siliciumcarbid, Zinndioxid, Rutil, Tonerde, Aluminiumsilikat, Magnesiumsilikat (Steatit), Zirkoniumsilikat, Cersilikat oder Mischungen dieser Trägermaterialien aufgebracht ist. Beispielsweise können mit dem erfindungsgemäßen Verfahren Schalenkatalysatoren mit chemischen Zusammensetzungen, wie sie in DE-A 2546268, EP-A 286448, DE-A 25 47 624, DE-A 2948163, EP-A 163231 oder DE-A 2830765 beschrieben sind und auf die hiermit bezüglich der Einzelheiten betreffend deren chemische Zusammensetzung Bezug genommen wird, hergestellt werden, wobei die erfindungsgemäß hergestellten Schalenkatalysatoren gegenüber diesen, auf herkömmliche Weise hergestellten Schalenkatalysatoren bezüglich Ausbeute, Selektivität und/oder Anfahrverhalten erheblich verbesserte katalytische Eigenschaften haben. Die Ursache hierfür ist nicht bekannt. Möglicherweise wird infolge der nach dem erfindungsgemäßen Verfahren erzeugten makroskopischen Struktur der Aktivmasseschale und/oder der variierten Aktivmassenzusammensetzung der Zugang des Edukts zu den katalytisch aktiven Zentren in dieser Aktivmasse erleichtert und gleichzeitig eine schnellere Abdiffusion des gebildeten Produkts aus der Aktivmasseschale ermöglicht, bevor dieses an den katalytisch aktiven Zentren weiter oxidativ abgebaut wird. Zusätzlich kann die geringe Flüchtigkeit mancher Hilfsmittel insbesondere der temporären Aktivitätsdämpfungsmittel dazu führen, daß diese nur langsam und vollständig erst im Verlaufe des Anfahrens des Reaktors bis zu dessen Vollastbetrieb entfernt werden und somit durch Blockierung von Aktivzentren die Katalysatoraktivität zunächst verringern. Bei diesen Versuchen der Erklärung der vorteilhaften Eigenschaften der erfindungsgemäßen Katalysatoren handelt es sich jedoch nur um eine reine Vermutung, da eine genaue Erfassung dieser Sachverhalte praktisch nicht möglich ist.

Die vorliegende Erfindung betrifft weiterhin ein Verfahren für die katalytische Gasphasenoxidation von aromatischen Kohlenwasserstoffen zu Carbonsäuren und/oder Carbonsäureanhydriden mit einem molekularen Sauerstoff enthaltenden Gas in einem Festbett bei erhöhter Temperatur und mittels einem oder mehreren in Schichten in Reaktor angeordneten Schalenkatalysatoren, auf deren Trägermaterial eine katalytisch aktive Metalloxide enthaltende Schicht schalenförmig aufgebracht ist, das dadurch gekennzeichnet ist, daß man mindestens einen Schalenkatalysator verwendet, der durch Beschichten eines Trägers mit einem Pulver, das aus einer Lösung und/oder einer Suspension der katalytisch aktiven Metalloxide und deren Vorläuferverbindungen oder dieser Vorläuferverbindungen alleine in An- oder Abwesenheit von Hilfsmitteln für die Katalysatorherstellung durch Sprühtrocknung oder Gefriertrocknung hergestellt worden ist, in An- oder Abwesenheit von Hilfsmitteln für die Katalysatorherstellung, nach oder ohne vorausgegangene Konditionierung des Pulvers und einer anschließenden Wärmebehandlung des so beschichteten Trägers zur Erzeugung der katalytisch aktivem Metalloxide, hergestellt worden ist.

Die erfindungsgemäß hergestellten Katalysatoren werden vorzugsweise zur Gasphasenoxidation aromatischer $C_6$- bis $C_{10}$-Kohlenwasserstoffe, wie Benzol, den Xylolen, insbesondere ortho-Xylol, Toluol, Naphthalin oder Durol (1,2,4,5-Tetramethylbenzol) zu Carbonsäuren und/oder Carbonsäureanhydriden wie Maleinsäureanhydrid, Phthalsäureanhydrid, Benzoesäure und/oder Pyromellithsäuredianhyrid, verwendet. Besonders bevorzugt wird das erfindungsgemäße Verfahren zur Herstellung von Phthalsäureanhydrid aus o-Xylol eingesetzt.

Zu diesem Zweck werden die erfindungsgemäß hergestellten Katalysatoren in von außen auf die Reaktionstemperatur, beispielsweise mittels Salzschmelzen, thermostatisierte Reaktionsrohre gefüllt und über die so bereitete Katalysatorschüttung das Reaktionsgas bei Temperaturen von im allgemeinen 300 bis 450°C, vorzugsweise von 320 bis 420°C und besonders bevorzugt von 340 bis 400°C und bei einem Überdruck von im allgemeinen 0,1 bis 2,5bar, vorzugsweise von 0,3 bis 1,5 bar mit einer Raumgeschwindigkeit von im allgemeinen 750 bis 5000 h$^{-1}$ geleitet.

Das dem Katalysator zugeführte Reaktionsgas wird im allgemeinen durch Vermischen von einem molekularen Sauerstoff enthaltenden Gas, das außer Sauerstoff noch geeignete Reaktionsmoderatoren und/oder Verdünnungsmittel, wie Dampf, Kohlendioxid und/oder Stickstoff, enthalten kann, mit dem zu oxidierenden, aromatischen Kohlenwasserstoff erzeugt, wobei das molekularen Sauerstoff enthaltende Gas im allgemeinen 1 bis 100 mol-%, vorzugsweise 2 bis 50 mol-% und besonders bevorzugt 10 bis 30mol-% Sauerstoff, 0 bis 30 mol-%, vorzugsweise 0 bis 10 mol-% Wasserdampf sowie 0 bis 50 mol-%, vorzugsweise 0 bis 1 mol-% Kohlendioxid, Rest Stickstoff, enthalten kann. Zur Erzeugung des Reaktionsgases wird das molekularen Sauerstoff enthaltende Gas, im allgemeinen mit 5g bis 120g je Nm$^3$ Gas, vorzugsweise 60 bis 120 g/Nm$^3$ Gas und besonders bevorzugt, 80 bis 115 g/Nm$^3$ Gas des zu oxidierenden, aromatischen Kohlenwasserstoffs beschickt.

Vorteilhaft wird die Gasphasenoxidation so durchgeführt, daß man zwei oder mehr Zonen, vorzugsweise zwei Zonen der im Reaktionsrohr befindlichen Katalysatorschüttung auf unterschiedliche Reaktionstemperaturen thermostatisiert, wozu beispielsweise Reaktoren mit getrennten Salzbädern, wie sie in DE-A 2201528 oder DE-A 2830765 beschrieben sind, eingesetzt werden können. Wird die Umsetzung in zwei Reaktionszonen durchgeführt, wie in DE-A 4013051 beschrieben, wird im allgemeinen die zum Gaseintritt des Reaktionsgases hin gelegene Reaktionszone, welche im allgemeinen 30 bis 80 mol-% des gesamten Katalysatorvolumens umfaßt, auf eine um 1 bis 20°C, vorzugsweise um 1 bis 10°C und insbesondere um 2 bis 8°C höhere Reaktionstemperatur als die zum Gasaustritt hingelegene Reaktionszone thermostatisiert. Alternativ kann die Gasphasenoxidation auch ohne Aufteilung in Temperaturzonen bei einer Reaktionstemperatur durchgeführt werden. Unabhängig von der Temperaturstrukturierung erweist sich das Verfahren als besonders vorteilhaft, wenn in den oben angegebenen Reaktionszonen der Katalysatorschüttung Katalysatoren eingesetzt werden, die sich in ihrer katalytischen Aktivität und/oder chemischen Zusammensetzung ihrer Aktivmasse unterscheiden. Vorzugsweise wird bei Anwendung zweier Reaktionszonen in der ersten, also zum Gaseintritt des Reaktionsgases hin gelegenen Reaktionszone, ein Katalysator eingesetzt, der im Vergleich zum Katalysator, welcher sich in der zweiten,.also zum Gasaustritt hin gelegenen Reaktionszone, befindet, eine etwas geringere katalytische Aktivität hat. Im allgemeinen wird in der ersten Reaktionszone ein in seiner katalytisch aktiven Masse mit Alkalimetalloxiden dotierter und in der zweiten Reaktionszone ein mit Phosphorverbindungen dotierter Katalysator eingesetzt. Die Zusammensetzung Alkalimetalloxid-dotierter Schalenkatalysatoren wird z.B. in DE-A 2436009, die Zusammensetzung Phosphor-haltiger Schalenkatalysatoren beispielsweise in DE-A 1769998 beschrieben, auf die im Hinblick auf weitere Einzelheiten der chemischen Zusammensetzung der Einfachheit halber Bezug genommen wird.

Im allgemeinen wird die Umsetzung durch die Temperatureinstellung so gesteuert, daß in der ersten Zone der größte Teil des im Reaktionsgas enthaltenen aromatischen Kohlenwasserstoffs bei maximaler Ausbeute umgesetzt wird. Vorzugsweise wird der aromatische Kohlenwasserstoff in der ersten Reaktionszone nahezu vollständig bei maximaler Ausbeute umgesetzt. Wird das erfindungsgemäße Verfahren unter Anwendung mehrerer Reaktionszonen, in denen sich unterschiedliche Katalysatoren befinden, durchgeführt, so können in allen Reaktionszonen, die erfindungsgemäß hergestellten Schalenkatalysatoren eingesetzt werden. Es können aber im allgemeinen bereits erhebliche Vorteile gegenüber herkömmlichen Verfahren erzielt werden, wenn nur in einer der Reaktionszonen der Katälysatorschüttung, beispielsweise der ersten Reaktionszone, ein erfindungsgemäß hergestellter Schalenkatalysator verwendet wird und in den übrigen Reaktionszonen, beispielsweise der zweiten oder letzten Reaktionszone auf herkömmliche Weise hergestellte Schalenkatalysatoren benutzt werden.

Die im erfindungsgemäßen Verfahren als Edukt eingesetzten Kohlen-wasserstoffe sind Bestandteile des Erdöls und werden aus diesen bei der Erdölraffination gewonnen.

Phthalsäureanhydrid (PSA) wird weltweit in großem Umfang hergestellt. PSA ist Ausgangsmaterial zur Herstellung von Weichmachern, wie Dioctylphthalat. Pyromellithsäuredianhydrid wird als Monomer zur Herstellung von Polyimiden benutzt.

Beispiele

Beispiel 1

Herstellung des Schalenkatalysators Ia - erfindungsgemäß

Aus einer Suspension, bestehend aus 250,0g Anatas, der eine BET-Oberfläche von 20m$^2$/g hatte (Analyse: Ti: 59,8 Gew.-%; S: 0,18 Gew.-%; P: 0,09 Gew.-%; Nb: 0,26 Gew.-%; K: 0,008 Gew.-%; Na: 0,02 Gew.-%; Zr: 0,007 Gew.-%; Pb: 0,011 Gew.-%; W: 0,02 Gew.-%; Sb: 0,01 Gew.-%), 18,1g Vanadyloxalat, 1,43g Cäsiumsulfat, 940g Wasser und 122g Formamid wurden in einem Sprühtrockner bei einer Gaseintrittstemperatur von 280°C und einer Gasaustrittstemperatur des Trocknungsgases (Luft) von 120°C 270g Pulver mit einer Partikelgröße von 3 bis 60μm für 90 Gew.-% des Pulvers hergestellt. 700g Steatit- (Magnesiumsilikat) Ringe mit einem äußeren Durchmesser von 8mm, einer Länge von 6mm und einer Wandstärke von 1,5mm wurden in einer Dragiertrommel bei 20°C während 20 Minuten mit 78g des Pulvers unter Zusatz von 56g eines 30 Gew.-% Wasser/70 Gew.-% Glycerin-Gemisches beschichtet. Anschließend wurde der so beschichtete Katalysatorträger bei 100°C getrocknet.

Das Gewicht der so aufgetragenen katalytisch aktiven Masse betrug nach Wärmebehandlung bei 400°C für 1/2h 10 Gew.-%, bezogen auf das Gesamtgewicht des fertigen Katalysators. Die aufgebrachte, katalytisch aktive Masse, also die Katalysatorschale, bestand aus 0,40 Gew.-% Cäsium (berechnet als Cs), 4.0 Gew.-% Vanadium (berechnet als V$_2$O$_5$) und 95,6 Gew.-% Titandioxid.

Beispiel 2

Herstellung des Schalenkatalysators Ib - erfindungsgemäß

Der Katalysator wurde wie in Beispiel 1 beschrieben, hergestellt, wobei jedoch 78 g des Pulvers mit 56g eines 43 Gew.-% Wasser/43 Gew.-% Glycerin/13 Gew.-% Oxalsäure-Gemisches auf den Träger aufgetragen wurden.

Beispiel 3

Herstellung des Schalenkatalysators Ic - erfindungsgemäß

Der Katalysator wurde, wie in Beispiel 1 beschrieben, hergestellt, wobei jedoch 78g des Pulvers zunächst mit 4,1g Melamin vermischt und anschließend auf den Träger aufgetragen wurden.

Beispiel 4

Herstellung des Schalenkatalysators Id - erfindungsgemäß

Der Katalysator wurde wie in Beispiel 1 beschrieben, hergestellt, wobei jedoch ein Sprühpulver mit geändertem Vanadium-Gehalt Verwendung fand. Dabei wurden zunächst 39g eines analog Beispiel 1 hergestellten Pulvers (Herstellung unter Verwendung von 22,6g Vanadyloxalat anstelle von 18,1g) mit 5,0 Gew.-% Vanadium-Gehalt (berechnet als V$_2$O$_5$) und daran anschließend 39g eines analog Beispiel 1 hergestellten Pulvers (Herstellung unter Verwendung von 13,6g Vanadyloxalat anstelle von 18,1g) mit 3,0 Gew.-% Vanadium-Gehalt (berechnet als V$_2$O$_5$) auf den Träger aufgetragen.

Beispiel 5

Herstellung des Schalenkatalysators Ie - Vergleich

700g Steatit-Ringe mit einem äußeren Durchmesser von 8mm, einer Länge von 6mm und einer Wandstärke von 1,5mm wurden in einer Dragiertrommel auf 210°C erhitzt und mit einer Suspension aus 250,0g Anatas einer Oberfläche von 20m$^2$/g, 18,1g Vanadyloxalat, 1,43g Cäsiumcarbonat, 940g Wasser und 122g Formamid solange besprüht, bis das Gewicht der auf diese Weise aufgetragenen Schicht 10% des Gesamtgewichts des fertigen Schalenkatalysators entsprach.

Die auf diese Weise aufgebrachte katalytisch aktive Masse, also die Katalysatorschale, bestand aus 0,40 Gew.-% Cäsium (berechnet als Cs), 4,0 Gew.-% Vanadium (berechnet als $V_2O_5$) und 95,6 Gew.-% Titandioxid. Zur Beschichtung wurden 580g Maische benötigt, d.h. ca. 31% der versprühten Maische gingen beim Auftragen durch Austrag verloren.

Beispiel 6

Herstellung von Katalysator II - nicht erfindungsgemäß

700g Steatit- (Magnesiumsilikat) Ringe mit einem äußeren Durchmesser von 8mm, einer Länge von 6mm und einer Wandstärke von 1,5mm wurden in einer Dragiertrommel auf 210° erhitzt und mit einer Suspension aus 400,0g Anatas mit einer BET-Oberfläche von 20m$^2$/g, 57,6g Vanadyloxalat, 14,4g Antimontrioxid, 2,5g Ammoniumhydrogenphosphat, 0.65g Cäsiumsulfat, 618g Wasser und 128g Formamid besprüht, bis das Gewicht der aufgetragenen Schicht 10,5% des Gesamtgewichts des fertigen Katalysators entsprach. Die auf diese Weise aufgebrachte katalytisch aktive Masse, also die Katalysatorschale, bestand aus 0,15 Gew.-% Phosphor (berechnet als P), 7,5 Gew.-% Vanadium (berechnet als $V_2O_5$), 3,2 Gew.-% Antimon (berechnet als $Sb_2O_3$), 0,1 Gew.-% Cäsium (berechnet als Cs) und 89,05 Gew.-% Titandioxid.

Beispiele 7-10

Herstellung von Phthalsäureanhydrid - erfindungsgemäß

In ein 3,85 m langes Eisenrohr mit einer lichten Weite von 25 mm wurden in den einzelnen versuchen jeweils eine Schüttung von 1,60 m Höhe eines der Katalysatoren Ia bis Id und darauf eine Schüttung von 1,30 m Höhe des Katalysators II gefüllt. Das mit den Katalysatoren befüllte Eisenrohr wurde so installiert, daß die Katalysatoren Ia bis Id jeweils zum Gaseintritt hin gelegen waren, also mit dem Eduktgas zuerst in Berührung kamen. Das Eisenrohr wurde mittels einer Salzschmelze thermostatisiert. Durch das Rohr wurden stündlich, von oben nach unten, 4,0 Nm$^3$ Luft mit einer kontinuierlich steigenden Beladung an 98,5 Gew.-%igem o-Xylol von anfangs 0 bis am Ende 80 g o-Xylol/Nm$^3$ Luft bei einem Überdruck von ca. 0,4 ($\pm$ 0,05) bar geleitet. Dabei wurden die in der folgenden Tabelle zusammengefaßten Ergebnisse erhalten.

| Beispiel: Katalysator-kombination | Salzbadtemperatur (°C) | max. PSA-Ausbeute (Gew.-%) | Anfahrzeit (d) |
|---|---|---|---|
| 7:Ia/II | 356 | 113,2 | 17 |
| 8:Ib/II | 351 | 114,2 | 24 |
| 9:Ic/II | 355 | 113,8 | 10 |
| 10:Id/II | 355 | 114,4 | 16 |

In der Tabelle bedeuten:
PSA - Phthalsäureanhydrid

$$\text{max. PSA-Ausbeute in Gew.-\%} = \frac{\text{PSA-Menge in Gramm} \cdot 100}{\text{Eingesetzte Menge an reinem o-Xylol in Gramm}}$$

Anfahrzeit - die zur Erhöhung der o-Xylol-Beladung von 0 auf 80 g o-Xylol/Nm$^3$ Luft benötigten Tage

Beispiel 11

Herstellung von PSA - Vergleich

Beispiel 11 wurde analog den Beispielen 7-10 mit dem Unterschied ausgeführt, daß an Stelle der erfindungsgemäßen Katalysatoren Ia-Id eine 1,60m hohe Schüttung des nach Beispiel 5 zum Vergleich hergestellten Katalysators Ie in das Reaktionsrohr gefüllt wurde. Die maximale Ausbeute an PSA, bezogen auf 100%iges o-Xylol, betrug bei 350°C 113,9 Gew.-% bei einer Anfahrzeit von 26d.

**Patentansprüche**

1. Verfahren zur Herstellung von Schalenkatalysatoren für die katalytische Gasphasenoxidation von aromatischen Kohlenwasserstoffen zu Carbonsäuren und/oder Carbonsäureanhydriden, auf deren Trägermaterial eine katalytisch aktive Metalloxide enthaltende Schicht schalenförmig aufgebracht ist, **dadurch gekennzeichnet, daß** aus einer Lösung und/oder einer Suspension der katalytisch aktiven Metalloxide und deren Vorläuferverbindungen oder allein dieser Vorläuferverbindungen in An- oder Abwesenheit von Hilfsmitteln für die Katalysatorherstellung durch Sprühtrocknung oder Gefriertrocknung zunächst ein Pulver hergestellt wird, das anschließend als Paste in An- oder Abwesenheit von Hilfsmitteln für die Katalysatorherstellung auf dem Träger nach oder ohne vorausgegangene Konditionierung, ohne vorausgegangene Wärmebehandlung zur Erzeugung der katalytisch aktiven Metalloxide schalenförmig aufgebracht und der auf diese Weise beschichtete Träger einer Wärmebehandlung zur Erzeugung der katalytisch aktiven Metalloxide unterzogen wird, wobei die durchschnittliche Schichtdicke der Aktivmasse der so hergestellten Schalenkatalysatoren 10 bis 250 µm beträgt.

2. Verfahren nach Anspruch 1, **dadurch gekennzeichnet, daß** zwei oder mehrere Pulver verschiedener Zusammensetzung, hergestellt nach Anspruch 1, in An- oder Abwesenheit von Hilfsmitteln für die Katalysatorherstellung auf dem Träger nach oder ohne vorausgegangene Konditionierung und ohne vorausgegangene Wärmebehandlung zur Erzeugung der katalytisch aktiven Metalloxide sequentiell schalenförmig aufgebracht werden und der auf diese Weise beschichtete Träger einer Wärmebehandlung zur Erzeugung der katalytisch aktiven Metalloxide unterzogen wird.

3. Verfahren nach den Ansprüchen 1 und 2, **dadurch gekennzeichnet, daß** der Lösung oder Suspension der katalytisch aktiven Metalloxide und deren Vorläuferverbindungen oder der Vorläuferverbindungen alleine oder dem aus dieser Lösung oder Suspension erzeugten Pulver als Hilfsmittel zur Katalysatorherstellung Bindemittel und/ oder Porenbildner und/oder temporäre Aktivitätsdämpfungsmittel zugesetzt werden.

4. Verfahren nach den Ansprüchen 1 bis 3, **dadurch gekennzeichnet, daß** der durch Beschichten des Trägers mit dem die katalytisch aktiven Metalloxide und deren Vorläuferverbindungen oder die Vorläuferverbindungen allein sowie gegebenenfalls Hilfsmittel zur Katalysatorherstellung enthaltenden Pulver hergestellte Schalenkatalysator vor seiner Verwendung einer Wärmebehandlung bei 250 bis 450°C unterzogen wird.

5. Verfahren nach den Ansprüchen 1 bis 4, **dadurch gekennzeichnet, daß** das die katalytisch aktiven Metalloxide und deren Vorläuferverbindungen oder die Vorläuferverbindungen alleine sowie gegebenenfalls Hilfsmittel zur Katalysatorherstellung enthaltende Pulver vor seiner Aufbringung auf den Träger auf eine Partikelgröße von 0,1 bis 100 µm konditioniert wird.

6. Verfahren nach den Ansprüchen 1 bis 5, **dadurch gekennzeichnet, daß** man als katalytisch aktive Metalloxide und als deren Vorläuferverbindungen neben Titandioxid des Anatastyps und Vanadiumverbindungen Oxide oder Vorläuferverbindungen der Oxide der Alkalimetalle, der Erdalkalimetalle, des Thalliums, Aluminiums, Zirkoniums, Eisens, Nickels, Kobalts, Mangans, Zinns, Silbers, Kupfers, Chroms, Molybdäns, Wolframs, Iridiums, Tantals, Niobs, Arsens, Antimons, Cers und/oder des Phosphors auf einen Träger aus Quarz, Porzellan, Magnesiumoxid, Siliciumcarbid, Zinnoxid, Rutil, Tonerde, Aluminiumsilikat, Magnesiumsilikat, Zirkoniumsilikat und/oder Cersilikat aufbringt.

7. Verfahren für die katalytische Gasphasenoxidation von aromatischen Kohlenwasserstoffen zu Carbonsäuren und/ oder Carbonsäureanhydriden mit einem molekularen Sauerstoff enthaltenden Gas in einem Festbett bei erhöhter Temperatur und mittels einem oder mehreren in Schichten im Reaktor angeordneten Schalenkatalysatoren auf deren Trägermaterial eine Schicht aus katalytisch aktiven Metalloxiden schalenförmig aufgebracht ist, **dadurch**

**gekennzeichnet, daß** man mindestens einen Schalenkatalysator verwendet, der nach dem Verfahren gemäß den Ansprüchen 1 bis 6 hergestellt worden ist.

8. Verfahren nach Anspruch 7, **dadurch gekennzeichnet, daß** man o-Xylol oder Naphthalin zu Phthalsäureanhydrid oxidiert.

9. Verfahren nach Anspruch 7, **dadurch gekennzeichnet, daß** man Durol zu Pyromellithsäuredianhydrid oxidiert.

10. Schalenkatalysatoren für die katalytische Gasphasenoxidation von aromatischen Kohlenwasserstoffen zu Carbonsäuren und/oder Carbonsäureanhydriden auf deren Trägermaterial aus Quarz, Porzellan, Magnesiumoxid, Siliciumcarbid, Zinnoxid, Rutil, Tonerde, Aluminiumsilikat, Magnesiumsilikat, Zirkoniumsilikat und/oder Cersilikat eine Schicht katalytisch aktiven Metalloxide schalenförmig aufgebracht ist, welche neben Titandioxid des Anatastyps und Vanadiumpentoxid Oxide der Alkalimetalle, der Erdalkalimetalle, des Thalliums, Aluminiums, Zirkoniums, Eisens, Nickels, Kobalts, Mangans, Zinns, Silbers, Kupfers, Chroms, Molybdäns, Wolframs, Iridiums, Tantals, Niobs, Arsens, Antimons, Cers und/oder des Phosphors enthält, **dadurch gekennzeichnet, daß** der Schalenkatalysator nach dem Verfahren gemäß den Ansprüchen 1 bis 6 hergestellt worden ist.

**Claims**

1. A process for producing coated catalysts for the catalytic gas-phase oxidation of aromatic hydrocarbons to give carboxylic acids and/or carboxylic anhydrides, on the support material of which a layer comprising catalytically active metal oxides is applied in the form of a shell, wherein a powder is first produced by spray drying or freeze drying from a solution and/or a suspension of the catalytically active metal oxides and their precursor compounds or these precursor compounds alone in the presence or absence of auxiliaries for catalyst production, the powder is subsequently applied in the form of a shell as a paste in the presence or absence of auxiliaries for catalyst production to the support after or without prior conditioning, without prior heat treatment to generate the catalytically active metal oxides and the support coated in this way is subjected to a heat treatment to generate the catalytically active metal oxides the average layer thickness of the active composition of the coated catalysts produced in this way is from 10 to 250 μm.

2. A process as claimed in claim 1, wherein two or more powders of different composition, produced as set forth in claim 1, are applied sequentially in the form of shells in the presence or absence of auxiliaries for catalyst production to the support after or without prior conditioning and without prior heat treatment to generate the catalytically active metal oxides and the support coated in this way is subjected to a heat treatment to generate the catalytically active metal oxides.

3. A process as claimed in claim 1 or 2, wherein binders and/or pore formers and/or temporary activity damping agents are added as auxiliaries for catalyst production to the solution or suspension of the catalytically active metal oxides and their precursor compounds or the precursor compounds alone or the powder produced from this solution or suspension.

4. A process as claimed in any of claims 1 to 3, wherein the coated catalyst produced by coating the support with the powder comprising the catalytically active metal oxides and their precursor compounds or the precursor compounds alone and, if required, auxiliaries for catalyst production is subjected before use to a heat treatment at from 250 to 450°C.

5. A process as claimed in any of claims 1 to 4, wherein the powder comprising the catalytically active metal oxides and their precursor compounds or the precursor compounds alone and, if required, auxiliaries for catalyst production is conditioned to a particle size of from 0.1 to 100 μm prior to its application to the support.

6. A process as claimed in any of claims 1 to 5, wherein the catalytically active metal oxides and their precursor compounds used are, apart from titanium dioxide in the anatase form and vanadium compounds, oxides or precursor compounds of the oxides of the alkali metals, the alkaline earth metals, thallium, aluminum, zirconium, iron, nickel, cobalt, manganese, tin, silver, copper, chromium, molybdenum, tungsten, iridium, tantalum, niobium, arsenic, antimony, cerium and/or phosphorus and are applied to a support comprising quartz, porcelain, magnesium oxide, silicon carbide, tin oxide, rutile, alumina, aluminum silicate, magnesium silicate, zirconium silicate and/or cerium silicate.

7. A process for the catalytic gas-phase oxidation of aromatic hydrocarbons to give carboxylic acids and/or carboxylic anhydrides using a gas comprising molecular oxygen in a fixed bed at elevated temperature and by means of one or more coated catalysts arranged in layers in the reactor, where the support material of the coated catalysts has a layer of catalytically active metal oxides applied to it in the form of a shell, wherein use is made of at least one coated catalyst which has been produced by a process as claimed in any of claims 1 to 6.

8. A process as claimed in claim 7, wherein o-xylene or naphthalene is oxidized to phthalic anhydride.

9. A process as claimed in claim 7, wherein durene is oxidized to pyromellitic dianhydride.

10. A coated catalyst for the catalytic gas-phase oxidation of aromatic hydrocarbons to give carboxylic acids and/or carboxylic anhydrides, the support material of which comprises quartz, porcelain, magnesium oxide, silicon carbide, tin oxide, rutile, alumina, aluminum silicate, magnesium silicate, zirconium silicate and/or cerium silicate and has applied to it, in the form of a shell, a layer of catalytically active metal oxides which comprises, apart from titanium dioxide in the anatase form and vanadium pentoxide, oxides of the alkali metals, the alkaline earth metals, thallium, aluminum, zirconium, iron, nickel, cobalt, manganese, tin, silver, copper, chromium, molybdenum, tungsten, iridium, tantalum, niobium, arsenic, antimony, cerium and/or phosphorus, wherein the coated catalyst has been prepared by a process as claimed in any of claims 1 to 6.

## Revendications

1. Procédé pour la préparation de catalyseurs à coquille pour l'oxydation catalytique en phase gazeuse d'hydrocarbures aromatiques en acides carboxyliques et/ou anhydrides d'acide carboxylique, sur le matériau support desquels on a appliqué une couche d'oxydes de métal catalytiquement actifs en forme de coquille, **caractérisé en ce qu'**on réalise d'abord une poudre à partir d'une solution et/ou d'une suspension des oxydes de métal catalytiquement actifs et leurs composés précurseurs ou uniquement de ces composés précurseurs, en présence ou en l'absence d'adjuvants pour la préparation du catalyseur, par séchage par pulvérisation ou lyophilisation, qui est ensuite appliquée en forme de coquille comme pâte, en présence ou en l'absence d'adjuvants pour la préparation du catalyseur sur le support, après ou sans conditionnement préalable, sans traitement thermique préalable pour l'obtention des oxydes de métal catalytiquement actifs et le support ainsi revêtu est soumis à un traitement thermique pour l'obtention des oxydes de métal catalytiquement actifs, l'épaisseur de couche moyenne de la masse active du catalyseur à coquille ainsi préparé étant de 10 à 250 µm.

2. Procédé selon la revendication 1, **caractérisé en ce que** deux ou plusieurs poudres de compositions différentes, préparées selon la revendication 1 sont appliquées séquentiellement en forme de coquille, en présence ou en l'absence d'adjuvants pour la préparation du catalyseur sur le support, après ou sans conditionnement préalable et sans traitement thermique préalable pour l'obtention des oxydes de métal catalytiquement actifs et le support ainsi revêtu est soumis à un traitement thermique pour l'obtention des oxydes de métal catalytiquement actifs.

3. Procédé selon les revendications 1 et 2, **caractérisé en ce qu'**on ajoute à la solution ou la suspension des oxydes de métal catalytiquement actifs et leurs composés précurseurs ou des composés précurseurs seuls ou à la poudre obtenue à partir de cette solution ou suspension, comme adjuvants pour la préparation du catalyseur, des liants et/ou des promoteurs de pores et/ou des agents de réduction temporaires de l'activité.

4. Procédé selon les revendications 1 à 3, **caractérisé en ce que** le catalyseur à coquille obtenu par revêtement du support avec la poudre contenant les oxydes de métal catalytiquement actifs et leurs composés précurseurs ou les composés précurseurs seuls ainsi que, le cas échéant, des adjuvants pour la préparation du catalyseur, est soumis avant son utilisation à un traitement thermique entre 250 et 450°C.

5. Procédé selon les revendications 1 à 4, **caractérisé en ce que** la poudre contenant les oxydes de métal catalytiquement actifs et leurs composés précurseurs ou les composés précurseurs seuls ainsi que, le cas échéant, des adjuvants pour la préparation du catalyseur, est soumise avant son application sur le support à un conditionnement pour obtenir une grosseur de particule de 0,1 à 100 µm.

6. Procédé selon les revendications 1 à 5, **caractérisé en ce qu'**on applique comme oxydes de métal catalytiquement actifs et comme leurs composés précurseurs, outre le dioxyde de titane du type anatase et des composés du vanadium, des oxydes ou des composés précurseurs des oxydes de métaux alcalins, de métaux alcalino-terreux, du thallium, de l'aluminium, du zirconium, du fer, du nickel, du cobalt, du manganèse, de l'étain, de l'argent, du cuivre, du chrome, du molybdène, du tungstène, de l'iridium, du tantale, du niobium, de l'arsenic, de l'antimoine, du cérium et/ou du phosphore sur un support en quartz, porcelaine, oxyde de magnésium, carbure de silicium, oxyde d'étain, rutile, alumine, silicate d'aluminium, silicate de magnésium, silicate de zirconium et/ou silicate de cérium.

7. Procédé pour l'oxydation catalytique en phase gazeuse d'hydrocarbures aromatiques en acides carboxyliques et/ou anhydrides d'acide carboxylique avec un gaz contenant de l'oxygène moléculaire dans un lit fixe à température élevée et au moyen d'un ou de plusieurs catalyseurs à coquille disposés en couches dans le réacteur, sur le matériau support desquels on a appliqué une couche d'oxydes de métal catalytiquement actifs en forme de coquille, **caractérisé en ce qu'**on utilise au moins un catalyseur à coquille qui a été préparé selon le procédé selon les revendications 1 à 6.

8. Procédé selon la revendication 7, **caractérisé en ce qu'**on oxyde du O-xylène ou du naphtalène en anhydride de l'acide phtalique.

9. Procédé selon la revendication 7, **caractérisé en ce qu'**on oxyde du durène en dianhydride de l'acide pyromellitique.

10. Catalyseurs à coquille pour l'oxydation catalytique en phase gazeuse d'hydrocarbures aromatiques en acides carboxyliques et/ou anhydrides d'acide carboxylique, sur le matériau support en quartz, porcelaine, oxyde de magnésium, carbure de silicium, oxyde d'étain, rutile, alumine, silicate d'aluminium, silicate de magnésium, silicate de zirconium et/ou silicate de cérium desquels on a appliqué une couche d'oxydes de métal catalytiquement actifs en forme de coquille, qui contient, outre le dioxyde de titane du type anatase et du pentoxyde de vanadium, des oxydes de métaux alcalins, de métaux alcalino-terreux, du thallium, de l'aluminium, du zirconium, du fer, du nickel, du cobalt, du manganèse, de l'étain, de l'argent, du cuivre, du chrome, du molybdène, du tungstène, de l'iridium, du tantale, du niobium, de l'arsenic, de l'antimoine, du cérium et/ou du phosphore, **caractérisé en ce que** le catalyseur à coquille a été préparé selon le procédé selon les revendications 1 à 6.